# EUROPÄISCHE PATENTSCHRIFT

(11) **EP 0 699 659 B1**
(45) Veröffentlichungstag und Bekanntmachung des Hinweises auf die Patenterteilung: **10.02.1999**
(21) Anmeldenummer: 95113008.7
(22) Anmeldetag: 18.08.1995
(51) Int. Cl.: C07C 263/20, B01D 3/10

(54) **Verfahren und Vorrichtung zur kontinuierlichen Abtrennung eines festen Rückstandes aus seiner Lösung im gerührten Gutbett**
Process and device for separating a solid residue from its solution in stirred material bed
Procédé et dispositif pour la séparation d'un résidu solide à partir de sa solution dans un lit de matière agité

(30) Priorität: 31.08.1994 DE 4430951
(43) Veröffentlichungstag der Anmeldung: 06.03.1996
(73) Patentinhaber: BAYER AG, 51368 Leverkusen (DE)
(72) Erfinder: Hetzel, Hartmut, Dr., D-50858 Köln (DE); Grenner, Dieter, Dr., D-51373 Leverkusen (DE); Ebner, Wolfgang, Dr., D-41540 Dormagen (DE); Biskup, Klaus, Dr., D-51373 Leverkusen (DE)

(56) Entgegenhaltungen:
- EP-A- 0 000 463
- EP-A- 0 548 685
- EP-A- 0 626 368
- DE-B- 2 012 294
- US-A- 2 889 257
- US-A- 3 140 305
- US-A- 4 913 771
- US-A- 5 183 540
- US-A- 5 349 082

## Beschreibung

Insbesondere bei Destillationsprozessen fallen im Sumpf im wesentlichen undefinierte, nach Isolation feste Rückstände an, die entsorgt werden müssen. Im Interesse der Handhabbarkeit werden diese festen Rückstände bei der Destillation nicht vollständig isoliert, sondern in Form einer Lösung in noch verdampfbaren Wertstoffen oder speziell zugesetzten Lösungsmitteln aus der Destillation ausgeschleust. Diese Rückstandslösung erfordert zur Gewinnung der verdampfbaren Wertstoffe und zur Rückgewinnung evtl. eingesetzter Lösungsmittel eine weitere Aufarbeitung. Die Aufarbeitung erfolgt im allgemeinen so, daß die Rückstandslösung auf die Verdampfungstemperatur für verdampfbare Wertstoffe und Lösungsmittel erhitzt wird, die verdampfenden Stoffe abgezogen werden, wobei ein fester, rieselfähiger Rückstand erhalten wird, der der weiteren Entsorgung wie Verbrennung oder Deponierung zugeführt wird. Verfahrenstechnisch problematisch ist, daß die Rückstandslösung während der Verdampfung der verdampfbaren Wertstoffe bzw. Lösungsmittel von einem flüssigen Zustand über einen Zustand mit zäher, klebriger Konsistenz zum festen Rückstand übergeht. Erfolgt die Verdampfung diskontinuierlich in gerührten Apparaten, kann dieser Übergang anhand der stark ansteigenden erforderlichen Rührerleistung beim Durchgang durch den zäh-klebrigen Zustand beobachtet werden. Während des zäh-klebrigen Zustandes ist ferner der Wärmeübergang auf die Rückstandsmasse (Zuführung der Verdampfungswärme) sowie die Durchmischung zur Erzeugung neuer Oberflächen mit hoher Konzentration an verdampfbaren Stoffen stark behindert.

Ein Beispiel für solcherart Destillationsrückstände sind Rückstände aus der Destillation bei der Herstellung von Isocyanaten, insbesondere Toluylendiisocyanat (TDI), einer Hauptkomponente zur Herstellung von Polyurethan. Die Herstellung von Isocyanaten erfolgt durch Phosgenierung von entsprechenden Aminen, wobei die Reaktanden in einem Lösungsmittel, zumeist ortho-Dichlorbenzol, zur Reaktion gebracht werden. Die Reaktion verläuft mit einer Ausbeute von ca. 96 bis 98 %, wobei als Nebenprodukte in erster Linie Isocyanat-Polymerisate gebildet werden.

Zur Gewinnung von reinen Isocyanaten wird die durch Phosgenierung erhaltene rohe Isocyanat-Lösung in mehreren Teilschritten destilliert. Als Sumpfprodukt der Destillationskolonnen fällt eine Rückstandslösung an, die im Falle der Herstellung von TDI etwa 5 bis 20 % polymeres TDI enthält. Aus dieser Rückstandslösung sind verdampfbare Anteile an TDI und Lösungsmittel zu verdampfen, um einen festen, rieselfähigen Rückstand zu erhalten.

Gemäß US-A 2 889 257 wurde bereits vorgeschlagen, solche Rückstandslösungen aus der Isocyanatproduktion dadurch aufzuarbeiten, daß die Rückstandslösung in einen inerte, hochsiedende Kohlenwasserstoffe bei einer Temperatur von 200 bis 350°C enthaltenden gerührten Behälter eingeführt werden, wobei die verdampfbaren Anteile der Rückstandslösung verdampfen und abgezogen werden und wobei feste Rückstandsteilchen in dem heißen Kohlenwasserstofföl gebildet werden. Das heiße Kohlenwasserstofföl wird zwischen dem gerührten Behälter und einem Wärmeaustauscher umgepumpt, wobei aus einem Teilstrom gravimetrisch die festen Teilchen ausgeschleust werden. Durch dieses an sich elegante Verfahren wird der Übergang durch den zäh-klebrigen Zustand der Rückstandslösung umgangen, jedoch ist das Verfahren apparativ aufwendig und erfordert zusätzlich in gewissen Abständen die Aufarbeitung des Kohlenwasserstoff-Öls.

Nach einem neueren Vorschlag gemaß EP-A 548 685 gelingt die Aufarbeitung diskontinuierlich im gerührten Behälter, in dem 5 bis 20 % Bitumen bezogen auf die Menge der Rückstandslösung bei einer Temperatur von 150 bis 280°C vorgelegt wird, dem dann kontinuierlich die Rückstandslösung zugeführt wird, wobei die verdampfbaren Bestandteile der Rückstandslösung bei einem Druck von 2 bis 30 mbar abgezogen werden. Nach Beendigung der Charge wird ein fester Rückstand in Form einer krümeligen, frei fließenden Masse erhalten. Dabei erfüllt die Bitumen-Vorlage offenbar folgende Aufgaben: Der Rührbehälter wird anfänglich mit einer ausreichenden Wärmekapazität zur Verdampfung der verdampfbaren Bestandteile der Rückstandslösung ausgestattet, so daß Temperaturschwankungen vermieden werden. Bei der anfänglichen Einführung von Rückstandslösung sorgt der Bitumen für eine schnelle Verteilung der Rückstandslösung im gerührten Behälter. Das Anbacken von festem Rückstand am Rührer und an den Behälterwänden wird vermieden. Ferner wird die Ausbildung kleiner Rückstandskrümel begünstigt. Allerdings wird das Problem des Übergangs durch den zäh-klebrigen Zustand, der eine erheblich erhöhte Rührleistung erfordert, nur wenig gemildert. Nachteilig ist ferner die hohe Bedienungsaufwand erfordernde diskontinuierliche Fahrweise, die lediglich durch den abwechselnden Betrieb mehrerer Rührbehälter quasi kontinuierlich ausgebildet werden kann.

Erfindungsgemäß wird nun vorgeschlagen, die Rückstandslösung gegebenenfalls nach Vermischung mit bis zu 20 Gew.-% Bitumen bezogen auf das Gewicht der Rückstandslösung, auf ein gerührtes Bett aus festem, körnigem Gut, das auf der für die Verdampfung der verdampfbaren Wertstoffe und/oder Lösungsmittel gehalten wird, aufzubringen.

Gegenstand der vorliegenden Erfindung ist demgemäß ein Verfahren zur Abtrennung eines nach Abtrennung festen Rückstandes aus einer Lösung des Rückstandes in verdampfbaren Wertstoffen und/oder Lösungsmitteln, gegebenenfalls unter Zusatz von bis zu 20 Gew.-% an hochsiedenden Kohlenwasserstoffen, die unter Verdampfungsbedingungen der Wertstoffe inert sind, Erhitzen der Mischung auf Verdampfungstemperatur unter Vakuum, wobei die Wertstoffe verdampfen, abgezogen und kondensiert werden, und wobei der Rückstand als rieselfähiger Feststoff anfällt, das dadurch gekennzeichnet ist, daß die Rückstandslösung auf ein bei Verdampfungstemperatur gehaltenes gerührtes Bett aus rieselfähigem, festem Gut aufgebracht wird.

Als rieselfähiges, festes Gut wird vorzugsweise fester Rückstand, der bei einer vorangegangenen Abtrennung gewonnen wurde, eingesetzt.

Als Rückstandslösung dient vorzugsweise der Destillationssumpf aus der Destillation der Isocyanat-Herstellung, insbesondere der TDI-Herstellung.

Als hochsiedender inerter Kohlenwasserstoff wird vorzugsweise Bitumen eingesetzt, im Zusammenhang mit der Aufarbeitung von Isocyanat-Destillationsrückständen besonders bevorzugt in einer Menge von 1 bis 10 Gew.-%, insbesondere bevorzugt in einer Menge von 1 bis 5 Gew.-%, bezogen auf die Menge der Rückstandslösung.

Das Verfahren ist jedoch nicht auf die Aufarbeitung des Destillationssumpfes aus der Isocyanat-Herstellung beschränkt, sondern ist allgemein für die Aufarbeitung von Lösungen bzw. Suspensionen durch Abtrennung verdampfbarer Bestandteile unter Erzeugung eines festen Rückstandes geeignet. Beispielhafte Einsatzgebiete für das erfindungsgemäße Verfahren sind die Aufarbeitung von Destillationsrückständen allgemein, von Keramiksuspensionen, von Pigmentsuspensionen, oder auch aus Molkeemulsionen, die bei der Käseherstellung anfallen. Dabei kann der als Rückstand erhaltene Feststoff auch der im Vordergrund des Interesse stehende, zu gewinnende Wertstoff sein, z.B. im Anschluß an Extraktionsprozesse.

Ohne Beschränkung der Allgemeinheit der Erfindung wird diese am Beispiel der Aufarbeitung des Destillationssumpfes aus der Isocyanatherstellung beschrieben.

Das erfindungsgemäße Verfahren kann diskontinuierlich (batchweise) oder kontinuierlich durchgeführt werden.

Bei batchweiser Verfahrensführung wird das rieselfähige, feste Gut in einem mit Rührer versehenen Behälter vorgelegt, der Behälter auf Verdampfungstemperatur für die verdampfbaren Stoffe gebracht, evakuiert. Nach Erreichen der Betriebsbedingungen für die Abtrennung wird die Rückstandslösung in den Behälter eingeleitet, wobei die verdampfbaren Stoffe über die Gasphase abgezogen werden. Die Einleitung der Rückstandslösung erfolgt dabei mit einer solchen Rate, daß die mittlere Bett-Temperatur konstant bleibt, d.h. nicht absinkt und die Leistungsaufnahme des Rührers nicht ansteigt.

Das Bett wird vorzugsweise wandgängig gerührt, so daß einerseits ein guter Wärmeübergang zwischen beheizter Behälterwand und dem Bett gewährleistet ist und andererseits eine möglichst vollständige Umwälzung des festen rieselfähigen Gutes im gesamten Rührbehälter zur Gewährleistung einer möglichst gleichmäßigen Wärmeverteilung im Behälter erfolgt. "Wandgängig" bedeutet dabei, daß der Rührer einen Abstand von 2 bis 20 mm aufweist.

Beim Aufbringen der Rückstandslösung auf das gerührte feste Bett wird die Rückstandslösung dabei so stark verteilt, daß die verdampfbaren Bestandteile bei der herrschenden Temperatur und dem Druck von vorzugsweise 2 bis 30 mbar, besonders bevorzugt 10 bis 20 mbar, spontan verdampfen, wobei der Übergang durch den zäh-klebrigen Zustand in so dünnen Filmen erfolgt, daß die Eigenschaften des Bettes nicht beeinflußt werden. Im Falle der Aufarbeitung von Rückstandslösungen aus der TDI-Destillation weist das gerührte Bett vorzugsweise eine Temperatur von 150 bis 280°C, besonders bevorzugt 180 bis 230°C auf.

Während der Einleitung der Rückstandslösung wird kontinuierlich fester Rückstand gebildet, so daß die Masse des Bettes vergrößert wird, bis die Kapazität des Behälters erreicht ist. Danach wird der Behälter soweit entleert, daß lediglich soviel Feststoff im Behälter verbleibt, wie als Vorlage für das Bett der nächsten Charge erforderlich ist.

Die Menge des im Rührbehälter vorgelegten rieselfähigen festen Gutes, d.h. die im Behälter nach Entleerung verbleibende Menge an festem Rückstand, beträgt vorzugsweise mindestens 10 %, besonders bevorzugt 15 bis 30 % der Behälterkapazität.

Vorzugsweise wird das erfindungsgemäße Verfahren kontinuierlich durchgeführt.

Die Durchführung des kontinuierlichen Verfahrens kann in einem geheizten achsial fördernden Schnecken- oder Schaufeltrockner mit Entgasungseinrichtung erfolgen. Am Eingang des Schneckentrockners wird erfindungsgemäß das das Bett bildende feste körnige Gut kontinuierlich eingebracht. In einem Abstand in Förderrichtung des Schneckentrockners, der zum Aufheizen des körnigen Gutes auf Verdampfungstemperatur ausreicht, wird kontinuierlich die Rückstandslösung zudosiert. Am Auslaßende des Schneckentrockners wird kontinuierlich der gebildete feste Rückstand ausgeschleust.

Um die erforderliche Menge an in den Schneckentrockner einzutragendem festem körnigen Gut (Bettvorlage) niedrig zu halten, ohne daß an der Zugabestelle für die Rückstandslösung in dem Schneckentrockner eine flüssige Phase gebildet wird, wird die Rückstandslösung vorzugsweise über die Länge des Schneckentrockners verteilt eingespeist. Die Menge des erforderlichen eingetragenen körnigen Gutes kann so bei 10 bis 30 Gew.-% des Schneckenaustrages gehalten werden.

Bevorzugt wird das kontinuierliche Verfahren im gerührten Behälter durchgeführt, wobei der Rührer in Form von den Behälter in radiale Segmente aufteilenden, wandgängigen Rührblättern ausgebildet ist, sodaß eine umlaufende Bewegung des Gutes gewährleistet ist. Die Einleitung der Rückstandslösung erfolgt außerhalb der Behälterachse. Der Austrag des festen Rückstandes erfolgt am Radius des Behälters an einer Stelle, die in Drehrichtung des Rührers weitestmöglich von der Einleitstelle für die Rückstandslösung entfernt ist. Vorzugsweise wird die Rückstandslösung über mehrere konzentrisch zur Rührerachse angeordnete Einleitstellen zugegeben.

Ein den Querschnitt des Behälters radial teilendes Rührblatt ist dabei vorzugsweise aus einer Vielzahl kleinerer, schräg angestellter Rührblätter aufgebaut, die sich gegenseitig überdecken.

Bevorzugt wird das Bett so gerührt, daß die Rückstandsteilchen im Durchschnitt eine angenähert toroidförmige Bewegungsbahn beschreiben. Eine solche toroidförmige Bewegungsbahn wird bei an den Querschnitt des Rührbehälters angepaßten Rührblättern dadurch begünstigt, daß der Boden des Rührbehälters in Form eines Klöpperbodens ausgestaltet ist. Besonders bevorzugt ist der Klöpperboden des Rührbehälters in der Mitte nach innen eingezogen, so daß der Rührbehälter in Form eines Rundtroges ausgebildet ist.

Die kontinuierliche Entnahme des festen Rückstandes und die Aufgabe der Rückstandslösung auf das Bett erfolgt bezüglich der Drehrichtung des Rührers vorzugsweise hintereinander in der genannten Reihenfolge und benachbart, so daß die Teilchen des Gutbettes von der Aufgabestelle der Rückstandslösung den vorzugsweise rundtrogförmigen Behälter bis zur Entnahme des festen Rückstands wendelförmig in Drehrichtung des Rührers durchwandern. Auf diese Weise erhalten die Rückstandsteilchen eine definierte Mindestaufenthaltszeit im Rührbehälter nachdem sie mit der Rückstandslösung in Kontakt gekommen sind.

Gegenstand der vorliegenden Erfindung ist auch eine Vorrichtung zur Durchführung des Verfahrens, enthaltend einen beheizten Rührbehälter mit wandgängigem Rührer, Mitteln zur Aufrechterhaltung eines Unterdruckes im Behälter, Mitteln zur kontinuierlichen Zuführung von Rückstandslösung zum Behälter und Mitteln zum Austrag von rieselfähigem festem Rückstand aus dem Behälter, wobei der Rührbehälter in Form eines Rundtroges ausgebildet ist.

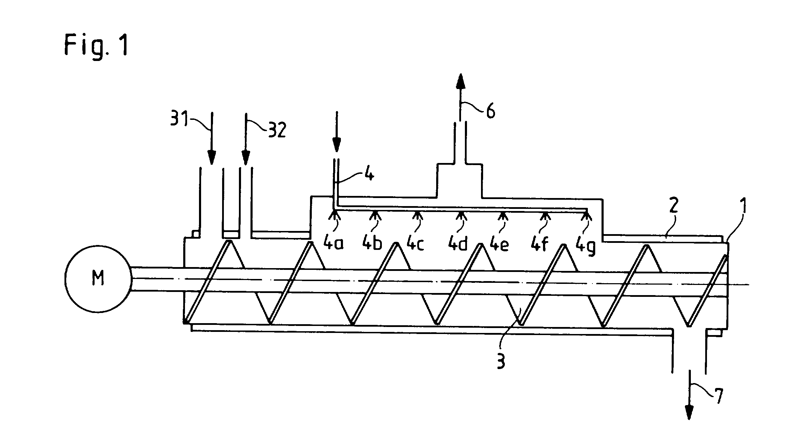

Weitere Einzelheiten der vorliegenden Erfindung werden anhand der beigefügten Fig. 1 bis 3 erläutert, die eine besonders bevorzugte Ausführungsform der vorliegenden Erfindung zeigen.

Fig. 1 zeigt einen für das erfindungsgemäße Verfahren geeigneten Schneckentrockner. Das Schneckengehäuse 1 weist einen Heizmantel 2 und eine wandgängige Schneckenelemente oder Schaufeln 3 auf. Pfeil 31 bezeichnet die Eingabestelle für das körnige Gut. Ferner kann ein Einlaß 32 für Bitumen vorgesehen sein. Die Einleitung der Rückstandslösung erfolgt über Leitung 4 mit Einleitdüsen 4 a bis 4 g. Entlang Pfeil 6 werden verdampfte Wertstoffe abgesaugt. Der Austrag des festen Rückstandes erfolgt bei 7.

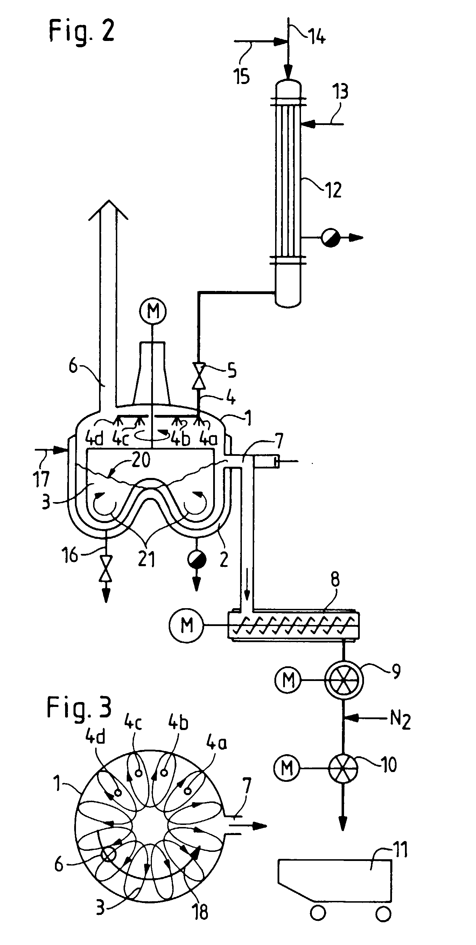

Fig. 2 zeigt den rundtrogförmigen Rührbehälter 1 mit Heizmantel 2 und wandgängigem Rührer 3. Das Bett aus festen Rückstandsteilchen, dessen Oberfläche durch Ziffer 20 angedeutet ist, führt unter der Drehbewegung des Rührers senkrecht zur Bewegung des Rührers 3 die durch Pfeile 21 angedeuteten kreisförmigen Bewegungen aus. Über Ventil 5 und Leitung 4 wird Rückstandslösung zugeführt und über Düsen 4a bis 4d auf das Gut aufgesprüht. Über Leitung 6 werden die verdampfenden Lösungsmittel und Wertstoffe mittels einer Vakuumpumpe abgezogen, in einen nicht gezeichneten Kondensator geleitet und anschließend destilliert. Kontinuierlich gebildeter fester Rückstand wird über den Auslaß 7 nach Abkühlung in der Kühl schnecke 8 über die Vakuumschleuse 9 ausgetragen. Gegebenenfalls ist eine zweite Schleuse 10 vorgesehen, wobei zwischen den Schleusen 9 und 10 Stickstoff eingeleitet werden kann, so daß der Durchtritt von Luftsauerstoff in den Rührbehälter 1 ausgeschlossen wird. Vor der Einführung der Rückstandslösung in den Rührbehälter kann ein Fallfilmverdampfer 12 mit Heizmittelzufuhr 13 vorgeschaltet sein, in den die Rückstandslösung über Leitung 14 zugeführt wird, wobei gegebenenfalls über Leitung 15 Bitumen zugesetzt wird. Der Fallfilmverdampfer wird insbesondere dann vorgeschaltet, wenn Rückstandslösungen mit einem Gehalt an nach Abtrennung festen Rückständen von weniger als 40 Gew.-% eingesetzt werden, da hierdurch die Kapazität des Rührbehälters erheblich gesteigert werden kann.

Fig. 3 zeigt eine Aufsicht auf das gerührte Bett zur Erläuterung der toroidförmigen Bewegungsbahnen 21 der Toroidteilchen. Der Auslaß 7 für den auszutragenden Rückstand ist zum Zulauf 4 für die Rückstandslösung benachbart angeordnet und bezüglich der Drehrichtung 18 des Rührers hintereinander.

## Patentansprüche

1. Verfahren zur Abtrennung eines nach Abtrennung festen Rückstandes aus einer Lösung des Rückstandes in verdampfbaren Wertstoffen und/oder Lösungsmitteln unter Zusatz von bis zu 20 Gew.-% hochsiedenden Kohlenwasserstoffen, die unter Verdampfungsbedingungen der Wertstoffe inert sind, Erhitzen der Mischung auf Verdampfungstemperatur unter Vakuum, wobei die Wertstoffe verdampfen, abgezogen und kondensiert werden und wobei der Rückstand als rieselfähiger Feststoff anfällt, dadurch gekennzeichnet, daß die Rückstandslösung auf ein bei Verdampfungstemperatur gehaltenes gerührtes Bett aus körnigem, festem Gut aufgebracht wird.

2. Verfahren nach Anspruch 1, dadurch gekennzeichnet, daß als festes körniges Gut vorher erzeugter fester Rückstand eingesetzt wird.

3. Verfahren nach Anspruch 1 oder 2, dadurch gekennzeichnet, daß dem gerührten Bett kontinuierlich rieselfähiger fester Rückstand entnommen wird.

4. Verfahren nach Anspruch 1 oder 2, dadurch gekennzeichnet, daß einem beheizten achsial fördernden Schnecken- oder Schaufeltrockner mit Absaugung am Kopf festes körniges Gut zugeführt wird und die Rückstandslösung auf das im Trockner geförderte Gut aufgebracht wird.

5. Verfahren nach einer der Ansprüche 1 bis 3, dadurch gekennzeichnet, daß die Rückstandsteilchen in einem Rührbehälter derart gerührt werden, daß sie im Durchschnitt eine angenäherte toroidförmige Bewegungsbahn beschreiben und die Rückstandslösung kontinuierlich aufgebracht wird.

6. Verfahren nach einem der Ansprüche 2 bis 3 oder 5, dadurch gekennzeichnet, daß die Entnahme des Rückstandes und Aufgabe der Rückstandslösung bezüglich der Drehrichtung des Rührers hintereinander und benachbart erfolgt.

7. Verfahren nach einem der Ansprüche 1 bis 6, dadurch gekennzeichnet, daß als Rückstandslösung der Destillationssumpf aus der Herstellung von TDI gegebenenfalls nach weiterer Aufkonzentrierung in einem Fallfilmverdampfer eingesetzt wird.

8. Vorrichtung zur Durchführung des Verfahrens nach einem der Ansprüche 1 bis 3 oder 5 bis 7, enthaltend einen beheizten Rührbehälter mit wandgängigem Rührer, Mitteln zur Aufrechterhaltung eines Unterdruckes im Behälter, Mitteln zur kontinuierlichen Zufuhr von Rückstandslösung zum Behälter und Mitteln zum kontinuierlichen Austrag von rieselfähigem, festem Rückstand aus dem Behälter, wobei der Rührbehälter in Form eines Rundtroges ausgebildet ist.

9. Vorrichtung nach Anspruch 8, wobei der Rührer in Form von wandgängigen, den Rührbehälter in axiale Sektoren aufteilenden Rührblättern ausgebildet ist.

## Claims

1. A process for separating a residue, which is solid after separation, from a solution of the residue in evaporable valuable materials and/or solvents with the addition of up to 20 wt.% of high-boiling hydrocarbons which are inert under evaporation conditions of the valuable materials, heating the mixture to evaporation temperature under vacuum, wherein the valuable materials evaporate, are drawn off and condensed, and wherein the residue is obtained as a free-flowing solid, characterised in that the residue solution is applied to a stirred bed of granular, solid material kept at evaporation temperature.

2. A process according to claim 1, characterised in that solid residue produced beforehand is used as solid granular material.

3. A process according to claim 1 or 2, characterised in that free-flowing solid residue is removed continuously from the stirred bed.

4. A process according to claim 1 or 2, characterised in that solid granular material is fed to a heated, axially conveying screw or paddle drier with suction at the head, and the residue solution is applied to the material conveyed in the drier.

5. A process according to one of claims 1 to 3, characterised in that the residue particles are stirred in a stirred vessel in such a way that they describe in general an approximately toroidal path and the residue solution is applied continuously.

6. A process according to one of claims 2 to 3 or 5, characterised in that the removal of the residue and the feeding of the residue solution takes place successively and adjacently in relation to the direction of rotation of the stirrer.

7. A process according to one of claims 1 to 6, characterised in that the distillation bottom product from the production of TDI is used as residue solution, optionally after further concentration in a falling film evaporator.

8. A device for carrying out the process according to one of claims 1 to 3 or 5 to 7, containing a heated stirred vessel with wall-sweeping stirrer, means for maintaining reduced pressure in the vessel, means for continuously feeding residue solution to the vessel, and means for continuously removing free-flowing solid residue from the vessel, wherein the stirred vessel is designed in the form of a round trough.

9. A device according to claim 8, wherein the stirrer is designed in the form of wall-sweeping blades dividing the stirred vessel into axial sectors.

## Revendications

1. Procédé pour la séparation d'un résidu solide issu de la séparation, d'une solution de résidu dans des matières valables et/ou dans des solvants aptes à être évaporés, avec addition d'hydrocarbures à points d'ébullition élevés jusqu'à concurrence de 20% en poids, qui sont inertes dans les conditions d'évaporation des matières valables, par chauffage du mélange à la température d'évaporation sous vide, dans lequel les matières valables s'évaporent, sont extraites et condensées, et dans lequel on obtient le résidu sous forme d'une substance solide s'écoulant librement, caractérisé en ce que la solution de résidu est appliquée sur un lit agité maintenu à la température d'évaporation, constitué par une matière solide granulée.

2. Procédé selon la revendication 1, caractérisé en ce qu'on met en oeuvre, à titre de matière granulée solide, un résidu solide obtenu au préalable.

3. Procédé selon la revendication 1 ou 2, caractérisé en ce qu'on prélève en continu du lit agité le résidu solide s'écoulant librement.

4. Procédé selon la revendication 1 ou 2, caractérisé en ce qu'on achemine de la matière granulée solide à un dessiccateur chauffé à palettes ou à vis sans fin à déplacement axial, dont la tête est équipée d'un dispositif d'aspiration sous vide, et on applique la solution du résidu sur la matière transportée dans le dessiccateur.

5. Procédé selon l'une quelconque des revendications 1 à 3, caractérisé en ce qu'on agite les particules de résidus dans un récipient d'agitation de telle sorte qu'elles décrivent en moyenne une trajectoire approximativement toroïdale et de telle sorte que la solution de résidu est appliquée en continu.

6. Procédé selon l'une quelconque des revendications 2 à 3 ou 5, caractérisé en ce que le prélèvement du résidu et l'addition de la solution de résidu se déroulent de manière successive et contiguë par rapport au sens de rotation de l'agitateur.

7. Procédé selon l'une quelconque des revendications 1 à 6, caractérisé en ce qu'on met en oeuvre, à titre de solution de résidu, le produit de bas de colonne de distillation issu de la préparation de TDI, le cas échéant après concentration ultérieure dans un évaporateur à film tombant.

8. Dispositif pour la mise en oeuvre du procédé selon l'une quelconque des revendications 1 à 3 ou 5 à 7 contenant un récipient d'agitation chauffé comportant un agitateur balayant les parois, des moyens pour maintenir une sous-pression dans le récipient, des moyens d'alimenter en continu la solution de résidu au récipient et des moyens pour évacuer en continu hors du récipient le résidu solide s'écoulant librement, le récipient d'agitation étant réalisé sous la forme d'une auge arrondie.

9. Dispositif selon la revendication 8, dans lequel l'agitateur est réalisé sous la forme de palettes d'agitation balayant les parois, qui subdivise le récipient d'agitation en secteurs axiaux.
